# EUROPEAN PATENT SPECIFICATION

(11) **EP 1 060 764 B1**
(45) Date of publication and mention of the grant of the patent: **29.03.2006**
(21) Application number: 99111099.0
(22) Date of filing: 18.06.1999
(51) Int. Cl.: A61N 5/10

(54) **Radiation source for endovascular radiation treatment**
Strahlungsquelle zur endovaskulären Bestrahlung
Source de rayonnement pour traitement endovasculaire

(43) Date of publication of application: 20.12.2000
(73) Proprietor: AEA Technology QSA GmbH, 38110 Braunschweig (DE)
(72) Inventor: Fritz, Eberhard, Dr., 38110 Braunschweig (DE); Phillipps, Gerd, Dr., 14169 Berlin (DE)
(74) Representative: Schwabe - Sandmair - Marx

(56) References cited:
- EP-A- 0 686 342
- WO-A-97/18012
- WO-A-97/19724
- WO-A-98/36790
- US-A- 4 584 991
- US-A- 4 940 452
- US-A- 5 683 345

## Description

The present invention relates to a radiation source for use in endovascular radiation treatment which radiation source comprises radiation emitting elements and is suitable for being delivered in a catheter to the selected site to be treated within the vascular system of a patient. The invention further relates to an apparatus for vascular radiation treatment using said radiation source,

### BACKGROUND OF THE INVENTION

Endovascular radiation treatment is the todays method of choice to prevent formation of scar tissue in a blood vessel which has been injured in various ways, for example, as trauma from surgical or diagnostic procedures. One area of the vascular system of particular concerns with respect to such injury is coronary arteries that are subjected to procedures for removing or reducing blockages due to plaques within the arteries. Partial and even complete blockage of the coronary arteries by the formation of an arteriosclerotic plaque is a well known and serious medical problem. Such blockages may be treated using arterectomy devices which mechanically remove the plaque, hot or cold lasers which vaporize the plaque, stents which hold the artery open and other devices and procedures well known in the art. The most common of them is the percutaneous transluminal coronary angioplasty, more commonly referred to as balloon angioplasty.

In this procedure a catheter having an inflatable balloon at its distal end is introduced into the coronary artery, the uninflated balloon is positioned at a stenotic site and the balloon is inflated. Inflation of the balloon disrupts and flattens the plaque against the arterial wall and stretches the arterial wall, resulting in enlargement of the intraluminal passageway and increased bloodflow. After such extension, the balloon is deflated and the balloon catheter removed.
Long term success of balloon angioplasty procedures is largly limited due to restenosis or re-closing of the intraluminal passageway through the artery by formation of scar tissue. Restenosis is experienced in approximately 30 to 50 % of the patients within six months after balloon angioplasty. Apparently restenosis is to a significant extend a natural healing response to the vessel injury caused by inflation of the angioplasty balloon.

Injury of a vessel typically initiates the bodies own natural repair and healing process. During the healing process, fibrin and plathelets rapidly accumulate in the endothelium and vascular smooth muscle cells proliferate and migrate into the intima. The formation of scar tissue by smooth muscle proliferation (hyperplasia) is believed to be a major contributor to restenosis following balloon angioplasty of the coronary artery.

Prior attempts to inhibit restenosis have included the use of various light therapies, chemotherapeutical agents, stents, arterectomy devices, hot and cold lasers and so on. The most promising approach to inhibit restenosis is the use of radiation therapy, i.e. the exposure of the restenotic site to ionizing or radioactive radiation.

Although radiation therapy in general has been applied advantageously, the devices available for delivery of radiation sources and the radiation sources themselves have certain drawbacks which limit their usefulness. Typically, the devices include a catheter, which is directed by way of a guide wire inserted therein to the site of treatment. The catheter is then used to internally direct the radiation source to the site of treatment and to retract the same after a predetermined treatment time.

One typical problem encountered with the catheter and/or the radiation source is related to stiffness of the source which is directly proportional to its length. Therefore, to allow the radiation source to travel along the bends of a vessel, typically shorter sources are used and the so-called "stepping treatment" is applied to achieve radiation treatment of the entire site. Since, however, very exact positioning is not possible in a constantly moving vessel such as coronary artery, long sources are desirable which allow for one-step treatment of the stenotic site in its entire length.

For example, US-A-5,833,593 discloses a source wire which is modified at its treatment end to receive a radioactive element. A plug seals the unmodified section of the source from the lumen of the modified segment which contains the radioactive element. Both ends of the source wire are sealed to prevent leakage of radioactivity. The source wire is then inserted in a catheter for guiding the same to the treatment site. The modified section (=container) itself is rigid and is only flexibly linked to the remainder, unmodified portion of the source.

From US-A-5,683,345 an apparatus and a method are known which apparatus includes an elongated flexible catheter tube having proximal and distal end portions with a lumen extending therebetween. One or more treating elements or seeds containing radioactive material are positionable within the lumen and are movable between the proximal and distal end portions under the force of liquid flowing through the lumen. The radiation source used according this document consists of individual treating elements which may be joined together to form a train of treating elements by use of several length of high tempered spring wire to prevent the treating elements from becoming too spaced apart while moving through the catheter.

The international publication WO 97/18012 discloses methods and devices for delivering low level radiation to inhibit neo-intimal hyperplasia following angioplasty or other intravascular procedures. In an exemplary method, a balloon (32) is inflated within a stenosed region of a blood vessel to produce a treated region. The balloon (32) is then deflated and a radioactive source (10) within a sleeve is aligned over the deflated balloon (32). The balloon (32) is again inflated at the treated region to engage the sleeve having the radioactive source (10) against the blood vessel within the treated region for from 1 minute to 49 minutes to deliver a sufficient dose of radiation to inhibit neo-intimal hyperplasia.

US-A-5 683 345 discloses a radiation source for use in endovascular radiation treatment comprising a train of seeds with radiation emitting element. The seeds are connected by spring wires.

Other typical drawbacks encountered with prior art radiation sources and devices for delivering the same to the site to be treated are related to the duration of exposure, controllability of the radiation exposure (dosage, homogeneity of treatment), the necessity to conduct a "stepping treatment", or difficulties in retracting the radiation source from the catheter and therefore the risk of undesirable exposure of both the patient and any medical personal handling the treatment device. It is the object of the invention to overcome these and other drawbacks of prior art radiation sources.

### SUMMARY OF THE INVENTION

These and other objects are achieved by the radiation source as defined in the appended claims. In a first aspect the invention relates to a radiation source for use in radiation treatment which comprises at least two treating elements (seeds) comprising a radiation emitting element and means for containment of said radiation emitting element, which radiation source is characterized in that the seeds are sequentially, directly and movably linked to each other either magnetically or mechanically as defined in claim 1. Thereby the seeds form a flexible radiation source, preferably a flexible elongated radiation source.

The means for containment of said radiation emitting element can be a capsule, preferably comprising a metal. Preferably the seeds have an elongated shape. In a preferred embodiment the central axis of the radiation source essentially parallels the elongated axis of the seeds.

According to a preferred embodiment the seeds are linked by magnetic forces to each other.

In a preferred embodiment the linkage is made mechanically and magnetically.

In mechanical linkage the seeds comprise male and female means for coupling which female means for coupling receive the male means for coupling of the following or proceeding seed in the radiation source to form a flexible joint. Preferably the male and female means for coupling are on opposing sides of the seed, even more preferably they are opposed to each other at the longitudinal ends on the end caps of the seed.

In another aspect, the invention relates to an apparatus for vascular radiation treatment, comprising (1) an elongated catheter having a proximal end portion, a distal end portion and a lumen extending therebetween for receiving a radiation source, (2) optionally a guide wire in a separate, second lumen, and (3) a radiation source as defined above.

Preferably the apparatus additionally comprises a x-ray fluoroscopy device for monitoring the radiation source. The apparatus may also comprise a containment vessel for storage of the radiation source and/or the individual seeds. In another embodiment the apparatus is an apparatus comprising a radiation source wherein the seeds are attached to each other by magnetic forces and the apparatus further comprises a magnetic means for guiding the radiation source.

The above radiation voice and apparatus can be used in a method for vascular radiation treatment comprising the steps of
(a) directing an elongated catheter having a proximal end portion, a distal end portion and a lumen extending therebetween for receiving a radiation source, to the selected site to be treated preferably by way of a guide wire in a separate lumen,
(b) introducing a radiation source into the catheter at its proximal end portion, which radiation source comprises one or more, preferably at least two treating elements (seeds) comprising a radiation emitting element and means for containment of said radiation emitting element, wherein said seeds are sequentially, directly and movably attached to each other and/or the transfer wire to form a flexible radiation source which can be moved through said lumen of the catheter,
(c) moving said radiation source to said distal end portion preferably by use of a transfer wire,
(d) maintaining said radiation source at said distal end portion for a determined period of time, and
(e) retracting said radiation source to the proximal end portion preferably by way of a transfer wire.

Preferably a radiation source as defined above is used.

Preferably moving and/or retracting in steps (c) and/or (e) is achieved by pushing or pulling the radiation source.

The seeds may be linked to each other by magnetic forces and the transfer wire comprises a magnet to magnetically effect said pulling of the radiation source in steps (c) and/or (e). In an alternative embodiment, an external magnetic field may be applied to move the radiation source comprising seeds linked to each other by magnetic forces.

The transfer wire may comprise a male or female means for coupling and a radiation source comprising such male and female means for coupling is linked to the transfer wire by engagement with the complementary means for coupling on the terminal seed thereof.

### BRIEF DESCRIPTION OF DRAWINGS

- Fig. 1: shows schematically a radiation source of the invention comprising magnetic seeds; Fig. 1a to 1c showing various shapes of seeds to be employed.
- Fig. 2a to 2c: schematically show a radiation source of the invention comprising seeds with male and female means for coupling.
- Fig. 3: is a schematic view of the catheter of the apparatus according to the invention.

### DETAILED DISCLOSURE OF THE INVENTION

In the following the invention will be described in detail referring to the attached drawings for illustration purposes. In these drawings like reference numerals refer to like parts. The term "radiation" is to be understood to relate to ionizing or radioactive radiation.

The radiation source for use in endovascular radiation treatment according to the invention comprises at least two treating elements, so-called seeds. These seeds comprise a radiation emitting element or radiation emitting core and means for containment of said radiation emitting element. The radiation source of the invention is characterized in that said seeds are sequentially, directly and movably linked to each other and thereby form a flexible radiation source of the desired length. The source may also be linked to a transfer corse.

The expression "directly" relates to a linkage which is achieved by direct engagement of the seeds themselves. The invention does not encompass linkage of the seeds by holding them together by use of external devices, containers or other fixing structures. Rather, the linkage of the seeds is obtained by forces exerted by the seeds themselves such as magnetic forces and/or a mechanical engagement of the seeds with each other wherein the seeds themselves form an active part of the joint. The expression "directly" does not exclude spacing members inserted between the seeds interrupting the chain of seeds by being interdisposed between the same, such as empty means for containment, non-radioactive magnetic particles, spheres and the like. A radiation source devoid of such spacing members is, however, preferred.

With the term "movably" as used herein, a linkage or joint between two seeds is meant which allows for any type of movement of the seeds relative to each other including rotational movement, bending or movement of one seed out of the axis of the central axis of the radiation source (the preceeding seeds still having their axis essentially parallel thereto), longitudinal movement of one seed away from the other without interruption or breaking the linkage, any type of vibration and so on, provided that the link between the seeds is not interrupted. The term "linked", "attached" and "joined" or the expression "linkage", "joined" and "coupling" are used interchangeably and refer to the interconnection between the seeds as defined above.

The radiation source of the present invention comprises at least two treating elements or seeds. Typically the number of seeds comprised in this radiation source is chosen to cover the desired length of the vessel to be treated. Preferably the radiation source will cover a number of seeds sufficient to provide a radiation source of at least 2 mm in length, preferably 10 to 50 mm in length, more preferably 20 to 40 mm in length. With the term "sequentially" it is meant that the seeds are linked to each other to form a chain of seeds. This does not exclude embodiments, wherein multiple e.g. 2, 3, 4 etc. smaller seeds are arranged in parallel and are then linked sequentially, directly and movably to the following duplett, triplett, quadruplett and so on.

Typically the individual seeds will have a length in the range of 1.0 to 10.0 mm, more preferably 1.5 to 4 mm and most preferred 2 to 3 mm.

Preferably the seeds are of the elongated, more preferably cylindrical shape and have an outer diameter of the means of containment thereof in the range of between 0.2 and 1.0 mm, preferably between 0.3 and 0.8 mm. Preferably the seeds comprise rounded (rounded edges of a generally flat end cap) or spherical end caps on one or both ends thereof.

The means for containment typically is a capsule. This capsule may be elongated, and may be hollow cylinder or tube comprising a first and a second end plug, but may have any shape suitable for forming seeds such as spheres, ellipsoids, doughnuts, cones, flat-end-tubes, disks, cubes etc., provided it comprises a cavity for receiving and enclosing said radiation emitting element and does not impair or inhibit movement of the seed in the catheter lumen.

Preferably the means for containment is a metallic capsule which comprises a metal selected from the group comprising stainless steel, Ag, Pt, Ti, Ni, Fe, Mn, Cr, Nb, Co, Au or there alloys, including mixtures thereof. It may also comprise any other suitable casing coated with one of these metals. More preferably the means for containment comprises a hollow cylindrical body having rounded or spherical end caps in one or both ends thereof, which may also form the above first and second end plug. More preferably the means of containment comprises a permanent magnetic material, most preferably Ni, Fe, stainless steel, Mn, Co and the like. According to another embodiment, the means for containment may also comprise a magnetizable material which is later magnetized by applying an external magnetic field.

The means for containment may also be formed from glass or plastics material such acrylics e.g. by coating a solid radiation emitting element to obtain a tight coating layer, provided it prevents leakage of radioactivity in the lumen of the catheter. It may further comprise a coating e.g. of teflon material or a similar low-friction material to reduce friction between the treating element or seed and the wall of the catheter lumen in which it moves.

The radiation emitting element comprised in said means for containment comprises any α-, *β*- and/or γ-emitting substance, preferably a pure *β* emitter and/or a *β*- and γ-emitting substance. Typically the radiation emitting element comprises one or more radioactive materials selected from the group comprising Cs¹³⁷, Co⁵⁷, Sr⁸⁹, Y⁹⁰, Au¹⁹⁸, Pd¹⁰³, Se⁷⁵, Sr⁹⁰, Ru¹⁰⁶, P³², Ir¹⁹², Re¹⁸⁸, W¹⁸⁸ and I¹²⁵ and other suitable nuclide(s).

The radioactive material may be contained in a solid such as metal, glass, foil or ceramics or in a free flowing form such as a powder or liquid or is dispersed in a fluid. Neither form nor state of the radioactive material is crucial, provided it allows for introducing the same in the means for containment and for secure containment.

The seeds are prepared by introducing the radiation emitting element into the means for containment made from the appropriate material and closing the same, e.g. by fixing the second end plug, e.g. by welding. The seeds may then be magnetized by applying an external magnetic field. Or the means for containment may be closed with appropriate end plugs having male and female coupling means.

The amount of radioactivity is typically in the range of 0.45 to 25,000 mCi per centimeter of vessel to be treated, depending on the radiation source used. The emitted radiation should be sufficient to deliver a desired dosage of from 100 to about 10,000 rads, preferably about 700 to 5,000 rads in a about 2 to 10 minutes to the tissue to be treated.

According to a preferred embodiment the radiation source is characterized in that the seeds are attached to each other by magnetic forces. According to this embodiment the seeds are held together by way of attracting magnetic forces e.g. forces exerted between the metallic capsules as means for containment of each seed. In this embodiment the means for containment is preferably made from a magnetic material such as Ni, Fe, Co, stainless steel, and Mn. According to another embodiment the means for containment may be made of a magnetizable material which is then magnetized by applying a magnetic field. The magnetic field can be applied immediately after production of the seeds or just before to use thereof. The magnetic field can be applied either by way of a permanent magnet or by an electromagnet.

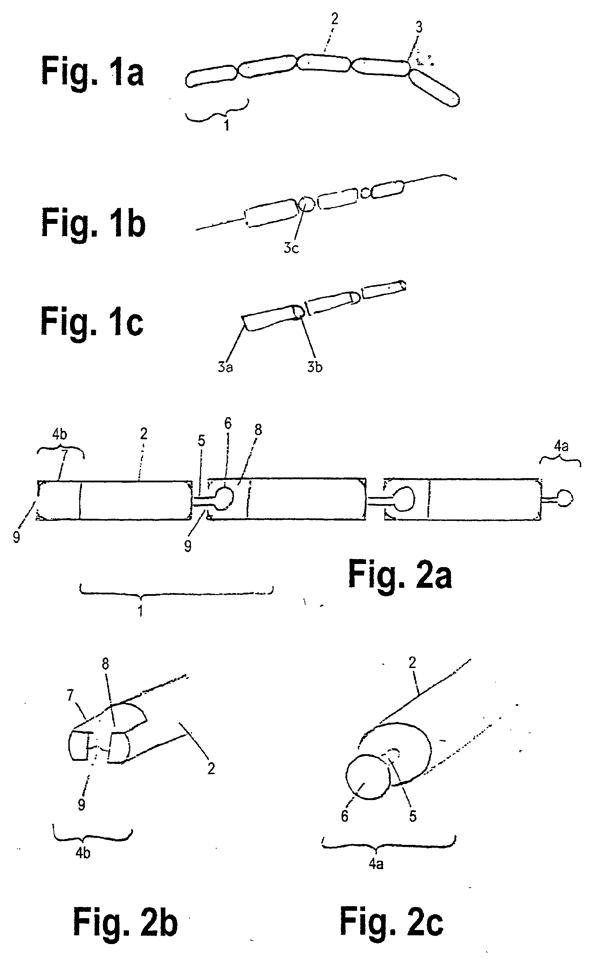

In a preferred embodiment shown in Fig. 1 the magnetic seeds (1) are attached in northsouth orientation. Thereby they form an elongated chain of seeds as the radiation source. More preferably the seeds of this chain comprise a means for containment (2) having rounded (3a) or spherical (3b) end caps (3) on one or both ends to allow smooth bending characteristics and improved magnetic adhesion of the seeds during bending of the chain.

In Fig. lb an embodiment is shown wherein the means of containments having rounded end caps (3a) are spaced apart by magnetic spheres (3c).

According to another embodiment of the radiation source according to the invention, the seeds are mechanically linked. The seeds may also be linked mechanically and magnetically to each other and/or the transfer wire.

The mode of mechanical linkage are seeds comprising male and female means for coupling which female means for coupling receive the male means for coupling of the following or proceeding seed in the radiation source to form a flexible joint. Preferably said male and female means for coupling are located on opposite sides of the seed, even more preferably they are opposed to each other at the longitudinal ends on the end caps of the means for containment of the seed. Providing the means for coupling on the end caps bears the advantage that they can be produced separately, e.g. by use of a laser and are only thereafter contacted with the tube containing the radiation emitting element and fixed to the tube e.g. by laser or point welding.

The male means for coupling may extend from the means for containment and may comprise a head and optionally a spacing member, whereas the female means for coupling in this case comprises a receiving section for the head. Said receiving section of the female means for coupling is preferably complementary to at least the head of the male means for coupling but still allows for movement of the joint such as rotational movement or bending or deflection from the central axis of the radiation source. Therefore the head of the male means for coupling preferably is in the form of a sphere.

In the preferred embodiment shown in Fig. 2a to 2c the male means for coupling (4a) comprises a spacing member (5) and a spherical head (6) and the receiving section of the female means for coupling (4b) is formed by extensions (7) of the means for containment (2), e.g. the capsule which extensions define a hollow space (8) having a recess (9) to receive the spacing member (5) when the head (6) of the male means for coupling is spaced in the hollow portion of said female means for coupling.

According to another embodiment of mechanical linkage, the male means for coupling is a hook and the female means for coupling is a second hook or a loop. In all cases both means for coupling may engage with each other or with a spacing member such as a ring, an empty means for containment or other member of a chain.

In case the coupling means and/or the containment means are made from a magnetic or magnetizable material, the seeds can be linked magnetically and mechanically.

All of the above linkages provide a chain of seeds or "train of treating elements" as a radiation source which allows for movement of said radiation source either by pushing or pulling at the terminal elements of the chain. The single seeds are linked to each other and preferably to the transfer wire with sufficient strength to allow such pulling movement without the radiation source being split up into its individual parts. On the other hand, the links between the seeds may be created or interrupted by the appropriate manipulation of each link. Thus, a radiation source of the desired length can be created and the length can be chosen appropriate for the intended use. At the same time the length of the source is not limited by its stiffness or rigidity due to the flexible joints. Thus, the radiation source of the invention allows for a one-step radiation treatment of elongated segments of a vessel.

Due to the movable link provided in the radiation source of the invention, this flexible radiation source can easily follow the bends and partitions of a blood vessel within the body to be treated. Apparently the radiation source of the invention is not limited to treatment of coronary restenosis, but may be used in any type of endovascular irradiation treatment e.g. in cancer therapy.

Due to direct engagement of the seeds with each other the source further allows for movement by pushing and pulling the chain of seeds. Thus, the source of the invention may be used in a catheter comprising only one central lumen for receiving the radiation source. Accordingly the seeds can be arranged in the central axis of the vessel to be treated to allow for uniform and homogenous irradiation of the surrounding tissue. This has to be considered an important aspect as radiation intensity decreases strongly with distance from the radiation source and an out of center location of the radiation source will result in unpredictable and non-controllable inhomogenities in the radiation field created therefrom. Thus, with an out of center arrangement of the radiation source inhomogeneous radiation of the surrounding tissue results. This is overcome by use of the present radiation source.

According to the present invention, there is further provided an apparatus for endovascular radiation treatment comprising (1) an elongated catheter having a proximal end portion, a distal end portion and a lumen extending therebetween for receiving a radiation source, (2) optionally a guide wire in a separate lumen and (3) a radiation source as disclosed above.

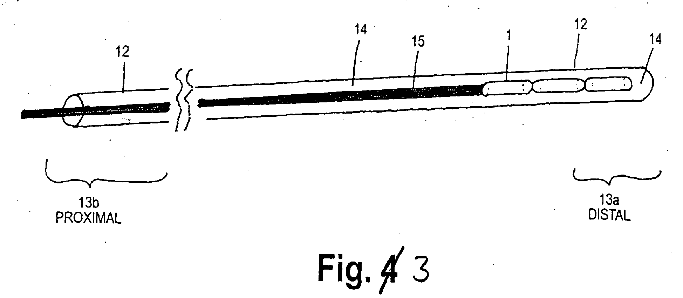

Referring to Fig. 3 the apparatus of the invention makes use of a catheter (12) which is typically made from nylon material, although other plastic or rubber material may be used as well. The outer diameter of the catheter is sized according to the intended application, e.g. 5 mm or smaller for use in treating the stenotic site of a coronary artery. The inner diameter of the lumen (14) extending between the distal end portion (13a) and the proximal end portion (13b) of the catheter is correspondingly sized to receive the treating elements or seeds (1) and is typically in the range of from about 0.2 to about 1.5 mm. Thus, to direct the flexible radiation source the internal diameter of the catheter is such that it permits movement of the seeds and helps to direct the radiation source. The catheter may have a coating, e.g. of Teflon or other suitable material to reduce friction upon movement of the radiation source. Likewise the catheter may be filled with a suitable liquid such as sterile water, PBS etc.

The catheter may not have sufficient strength or torsional rigidity for insertion along a lengthy serpentine vascular path and may then require use of a guide wire which ist then arranged in a separate lumen. Typically angioplasty procedures result in a distance between the percutaneous entryport and the coronary artery of approximately 90 to 120 cm, the length of the catheter corresponding thereto.

To assist in positioning the distal end portion (13a) of the catheter (12) at the desired location or site to be treated, the catheter may be advanced over a guide wire (15) that is preinserted to the desired location in the manner well known in the art. The guide wire is one commonly used in prior art and can be made from any suitable type of metal, preferably memory-resistant metals, i.e. materials that can accept up to a 1 % strain with less than a 1 % permanent alteration in its original configuration. Preferred materials include nickel-titanium alloys such as Nitinol or aluminum alloys such as Tinal alloy BB. In the apparatus of the invention, a separate wire is used for moving said radiation source which is the so-called transfer wire. This transfer wire can be made from the same materials as the guide wire and is preferably mechanically and/or magnetically linked to the radiation source. The guide wire is used for directing the catheter only.

The apparatus of the invention may further comprise a containment vessel for a storage of the radiation source and/or the individual seeds and for shielding the patient to be treated and the medical personal from exposure to irradiation during introduction and retraction of the catheter. The containment vessel preferably is in flow communication with the catheter, although it can be constructed as a separate or separable part to allow for separate storage and/or disposal.

The apparatus of the invention may further comprise a x-ray fluoroscopy device for monitoring the radiation source as, for example, described in US-A-5,833,593. This allows for exact positioning of the radiation source and, thus, for precise control of the treatment site.

Finally, the apparatus of the invention may comprise a magnetic means for guiding or moving the radiation source, in case the radiation source is created from magnetic seeds.

The above radiation soince and apparatus may be used in a method for vascular radiation treatment comprising the steps of
(a) directing an elongated catheter having a proximal end portion, a distal end portion and a lumen extending therebetween for receiving a radiation source, to the selected site to be treated preferably by way of a guide wire in a separate lumen,
(b) introducing a radiation source into the catheter at its proximal end portion, which radiation source comprises one or more, preferably at least two treating elements (seeds) comprising a radiation emitting element and means for containment of said radiation emitting element, wherein said seeds are sequentially, directly and movably attached to each other and/or to the transfer wire, and which can be moved through said lumen of the catheter,
(c) moving said radiation source to said distal end portion preferably by way of a transfer wire,
(d) maintaining said radiation source at said distal end for a determined period of time, and
(e) retracting said radiation source to the proximal end portion preferably by way of a transfer wire.

Preferably the radiation sources disclosed above are used in the method of the invention.

The steps of moving and/or retracting (c) and/or (e) can be achieved by pushing or pulling the radiation source.

More in detail, movement in step (c) may be achieved by pushing and said movement or retracting in step (e) is achieved by pulling said radiation source. For doing so, the radiation source may be linked to a transfer wire at its proximal end. In this embodiment the radiation source is introduced in the catheter lumen at its proximal end and pushed by use of the transfer wire to its distal end. After the predetermined treatment time, the radiation source is retracted by pulling out the transfer wire from the catheter. Alternatively, the radiation source may be engaged with the transfer wire at its distal end and may be pulled by said guide wire to the distal end of the catheter and pushed back to the proximal end portion during retracting of the source.

In case of a radiation source comprising magnetic seeds, movement of said radiation source in steps (c) and/or (e) may be achieved by applying an external magnetic field. Alternatively in this case the transfer wire may comprise a magnet to magnetically push or pull the radiation source in step (c) and/or (e). In the preferred embodiment the transfer wire itself is magnetic.

In case of the seeds being linked to each other by male and female means for coupling, the transfer wire may comprise a male or female means for coupling and the radiation source may be linked to the transfer wire at its distal or proximal end through the complementary means for coupling on the terminal seed thereof.

Separate wires are used for moving said radiation source (transfer wire) and for directing the catheter (guide wire).

Due to the use of a catheter having a single lumen for receiving the radiation source only, the inner diameter of said lumen can be increased as compared to catheters comprising several of such lumens. Accordingly, larger seeds may be used. This allows for including higher radiation dosages in each single seed. Use of a single lumen further allows for a central arrangement of the catheter and thus of the radiation source within the vessel. Thereby uniform and homogeneous irradiation of the surrounding tissue is achieved. Due to the seeds being directly linked to each other and due to flexible linkage, no gaps in the irradiated field occur and thus the radiation source needs not be moved during treatment i.e. no "stepping treatment" is required to obtain the homogeneous radiation over the entire segment of the vessel to be treated. This further improves control of the treatment.

Although being described with respect to the preferred embodiments above, this description is not to be considered limited thereto and the skilled worker will appreciate the possibility of several variations of the invention as defined in the appending claims without deviating from its scope.

## Claims

1. Radiation source for use in endovascular radiation treatment which comprises at least two seeds comprising a radiation emitting element and means for containment of said radiation emitting element, wherein said seeds are sequentially, directly and movably linked to each other **characterised in that** the linkage is made (a) by magnetic forces or (b) mechanically by male and female means for coupling, which female means for coupling receive the male means for coupling of the following or preceding seed in the radiation source to form a flexible joint.

2. Radiation source of claim 1, wherein the means for containment is a capsule.

3. Radiation source of claims 1 and 2, wherein said means for containment comprises a metal selected from the group consisting of stainless steel, Ag, Pt, Ti, Ni, Fe, Mn, Cr, Nb, Co, Au or their alloys or a casing coated with these metals.

4. Radiation source of one of claim 1 to 3, wherein the seeds have an elongated shape.

5. Radiation source of claim 4, wherein the central axis of the radiation source essentially parallels the elongated axis of the seeds.

6. Radiation source of one of claim 1 to 5, wherein radiation emitting element comprises any α-, β- or γ-emitting substance.

7. Radiation source of claim 6, wherein radiation emitting element comprises one or more radioactive materials selected from the group consisting of Cs¹³⁷, Co⁵⁷, Sr⁸⁹, Y⁹⁰, Au¹⁹⁸, Pd¹⁰³, Se⁷⁵, Sr⁹⁰, Ru¹⁰⁶, P³², Ir¹⁹², Re¹⁸⁸, W¹⁸⁸ and I¹²⁵.

8. Radiation source according to claims 1 to 7, wherein the seeds are linked by magnetic forces to each other.

9. Radiation source of claim 8, wherein said means for containment comprises a magnetizable or magnetic material.

10. Radiation source of claims 1 to 9, wherein the seeds have rounded or spherical end caps on one or both ends.

11. Radiation source of claim 13, wherein male the seeds are linked mechanically and magnetically.

12. Radiation source according to claim 1, wherein the seeds are mechanically linked and comprise male and female means for coupling, which female means for coupling receive the male means for coupling of the following or preceding seed in the radiation source to form a flexible joint.

13. Radiation source of claim 12, wherein male and female means for coupling are on opposing sides of the seed.

14. Radiation source of claims 12 or 13, wherein the male means for coupling comprises a head and the female means for coupling comprises a receiving section for the head.

15. Radiation source of claims 12 or 13, wherein the male means for coupling is a hook and the female means for coupling is a second hook or a loop.

16. Radiation source of claims 12 to 14, wherein the male means for coupling comprises a spacing member and a spherical head and the receiving section of the female means for coupling is formed by extensions of the means for containment defining a hollow space having a recess to receive the spacing member when the head of the male means for coupling is placed in the hollow portion of said female means for coupling.

17. Radiation source according to claim 1, wherein the seeds are further linked to a transfer wire.

18. Apparatus for endovascular radiation treatment, comprising an elongated catheter having a proximal end portion, a distal end portion and a lumen extending there-between for receiving a radiation source, and a radiation source as defined in claim 1.

19. Apparatus of claim 18, comprising a containment vessel for the radiation source and the individual seeds.

20. Apparatus of claims 18 to 19, comprising a x-ray fluoroscopy device.

21. Apparatus of claims 18 to 20, comprising a magnetic means.

22. Apparatus of claims 18 to 21, further comprising a guide wire in a separate lumen.

## Patentansprüche

1. Strahlungsquelle zur Verwendung bei einer endovaskulären Strahlungsbehandlung, die mindestens zwei Radioisotopenpräparate umfasst, die ein strahlungsabgebendes Element und ein Mittel zur Einschließung des strahlenabgebenden Elements umfassen, wobei die Radioisotopenpräparate sequentiell, direkt und bewegbar miteinander verbunden sind, **dadurch gekennzeichnet, dass** die Verbindung (a) durch Magnetkräfte oder (b) mechanisch mittels Einsteck- und Aufnahmemitteln zum Koppeln erfolgt, wobei die Aufnahmemittel zum Koppeln die Einsteckmittel zum Koppeln des folgenden oder vorhergehenden Radioisotopenpräparats in der Strahlungsquelle zur Bildung eines flexiblen Gelenks aufnehmen.

2. Strahlungsquelle nach Anspruch 1, wobei das Mittel zur Einschließung eine Kapsel ist.

3. Strahlungsquelle nach Anspruch 1 und 2, wobei das Mittel zur Einschließung ein Metall, ausgewählt aus der Gruppe, bestehend aus rostfreiem Stahl, Ag, Pt, Ti, Ni, Fe, Mn, Cr, Nb, Co, Au oder ihren Legierungen, oder ein mit diesen Metallen beschichtetes Gehäuse umfasst.

4. Strahlungsquelle nach einem der Ansprüche 1 bis 3, wobei die Radioisotopenpräparate eine längliche Gestalt aufweisen.

5. Strahlungsquelle nach Anspruch 4, wobei die zentrale Achse der Strahlungsquelle im Wesentlichen parallel zur langen Achse der Radioisotopenpräparate verläuft.

6. Strahlungsquelle nach einem der Ansprüche 1 bis 5, wobei das strahlungsabgebende Element eine beliebige α-, β- oder γ-abgebende Substanz umfasst.

7. Strahlungsquelle nach Anspruch 6, wobei das strahlungsabgebende Element eines oder mehrere radioaktive Materialien umfasst, die ausgewählt sind aus der Gruppe, bestehend aus Cs¹³⁷, Co⁵⁷, Sr⁸⁹, Y⁹⁰, Au¹⁹⁸, Pd¹⁰³, Se⁷⁵, Sr⁹⁰, Ru¹⁰⁶, P³², Ir¹⁹², Re¹⁸⁸, W¹⁸⁸ und I¹²⁵.

8. Strahlungsquelle nach den Ansprüchen 1 bis 7, wobei die Radioisotopenpräparate durch magnetische Kräfte miteinander verbunden sind.

9. Strahlungsquelle nach Anspruch 8, wobei das Mittel zur Einschließung, ein magnetisierbares oder magnetisches Material umfasst.

10. Strahlungsquelle nach den Ansprüchen 1 bis 9, wobei die Radioisotopenpräparate gerundete oder kugelförmige Endkappen an einem oder beiden Enden aufweisen.

11. Strahlungsquelle nach Anspruch 1, wobei die Radioisotopenpräparate mechanisch und magnetisch verbunden sind.

12. Strahlungsquelle nach Anspruch 1, wobei die Radioisotopenpräparate mechanisch verbunden sind und Einsteck- und Aufnahmemittel zum Koppeln umfassen, wobei die Aufnahmemittel zum Koppeln die Einsteckmittel zum Koppeln des folgenden oder vorhergehenden Radioisotopenpräparats in der Strahlungsquelle zur Bildung eines flexiblen Elements aufnehmen.

13. Strahlungsquelle nach Anspruch 12, wobei sich die Einsteck- und Aufnahmemittel zum Koppeln an entgegengesetzten Seiten des Radioisotopenpräparats befinden.

14. Strahlungsquelle nach Anspruch 12 oder 13, wobei die Einsteckmittel zum Koppeln einen Kopf aufweisen und die Aufnahmemittel zum Koppeln einen Aufnahmebereich für den Kopf aufweisen.

15. Strahlungsquelle nach Anspruch 12 oder 13, wobei das Einsteckmittel zum Koppeln ein Haken ist und das Aufnahmemittel zum Koppeln ein zweiter Haken oder eine Schleife ist.

16. Strahlungsquelle nach den Ansprüchen 12 bis 14, wobei das Einsteckmittel zum Koppeln ein Abstandselement und einen kugelförmigen Kopf aufweist und der Aufnahmebereich des Aufnahmemittels zum Koppeln mittels Verlängerungen des Mittels zur Einschließung gebildet ist, das einen Hohlraum mit einer Aussparung bildet, um das Abstandselement aufzunehmen, wenn der Kopf des Einsteckmittels zum Koppeln in dem hohlen Bereich des Aufnahmemittels zum Koppeln verbracht ist.

17. Strahlungsquelle nach Anspruch 1, wobei die Radioisotopenpräparate des weiteren mit einem Übertragungsdraht verbunden sind.

18. Vorrichtung zur endovaskulären Strahlungsbehandlung, umfassend einen länglichen Katheter mit einem proximalen Endbereich, einem distalen Endbereich und einem sich dazwischen erstreckenden Lumen zur Aufnahme einer Strahlungsquelle und eine Strahlungsquelle wie in Anspruch 1 definiert.

19. Vorrichtung nach Anspruch 18, umfassend ein Einschließungsgefäß für die Strahlungsquelle und die einzelnen Radioisotopenpräparate.

20. Vorrichtung nach den Ansprüchen 18 bis 19, umfassend eine Röntgenfluoroskopievorrichtung.

21. Vorrichtung nach den Ansprüchen 18 bis 20, umfassend ein Magnetmittel.

22. Vorrichtung nach den Ansprüchen 18 bis 21, des weiteren umfassend einen Führungsdraht in einem separaten Lumen.

## Revendications

1. Source de rayonnement pour l'utilisation dans le traitement par rayonnement endovasculaire, qui comprend au moins deux germes comprenant un élément émetteur de rayonnement et des moyens pour le confinement dudit élément émetteur de rayonnement, dans laquelle lesdits germes sont reliés en succession, directement et de façon mobile l'un à l'autre, **caractérisée en ce que** la liaison est effectuée (a) par des forces magnétiques ou (b) de façon mécanique par des moyens mâles et femelles pour le couplage, les moyens femelles servant à recevoir pour le couplage les moyens mâles pour le couplage du germe suivant ou précédent dans la source de rayonnement afin de former un raccord souple.

2. Source de rayonnement selon la revendication 1, dans laquelle les moyens pour le confinement sont une capsule.

3. Source de rayonnement selon les revendications 1 et 2, dans laquelle lesdits moyens pour le confinement comprennent un métal sélectionné parmi le groupe comprenant l'acier inoxydable, Ag, Pt, Ti, Ni, Fe, Mn, Cr, Nb, Co, Au, ou leurs alliages ou une enceinte revêtue de ces métaux.

4. Source de rayonnement selon l'une des revendications 1 à 3, dans laquelle les germes ont une forme allongée.

5. Source de rayonnement selon la revendication 4, dans laquelle l'axe central de la source de rayonnement est essentiellement parallèle au grand à l'axe des germes.

6. Source de rayonnement selon l'une des revendications 1 à 5, dans lequel l'élément émetteur de rayonnement comprend l'une quelconque parmi des substances émettrices α, β ou Y.

7. Source de rayonnement selon la revendication 6, dans laquelle l'élément émetteur de rayonnement comprend un ou plusieurs matériaux radioactifs sélectionnés parmi le groupe comprenant Cs¹³⁷, Co⁵⁷, Sr⁸⁹, Y⁹⁰, Au¹⁹⁸, Pd¹⁰³, Se⁷⁵, Sr⁹⁰, Ru¹⁰⁶, P³², Ir¹⁹², Re¹⁸⁸, W¹⁸⁸ et I¹²⁵.

8. Source de rayonnement selon les revendications 1 à 7, dans laquelle les germes sont reliés entre eux par des forces magnétiques.

9. Source de rayonnement selon la revendication 8, dans laquelle lesdits moyens pour le confinement comprennent un matériau magnétisable ou magnétique.

10. Source de rayonnement selon les revendications 1 à 9, dans laquelle les germes comportent des capuchons d'extrémité arrondis ou sphériques sur une ou sur les deux extrémités.

11. Source de rayonnement selon la revendication 13, dans laquelle les germes sont reliés mécaniquement et magnétiquement.

12. Source de rayonnement selon la revendication 1, dans laquelle les germes sont reliés mécaniquement et comprennent des moyens mâles et femelles pour le couplage, les moyens femelles servant à recevoir pour le couplage les moyens mâles pour le couplage du germe suivant ou précédent dans la source de rayonnement afin de former un raccord flexible.

13. Source de rayonnement selon la revendication 12, dans laquelle les moyens mâles et femelles pour le couplage se trouvent sur des côtés opposés du germe.

14. Source de rayonnement selon les revendications 12 ou 13, dans laquelle les moyens mâles pour le couplage comprennent une tête et les moyens femelles pour le couplage comprennent une section de réception pour la tête.

15. Source de rayonnement selon les revendications 12 ou 13, dans laquelle les moyens mâles pour le couplage sont un crochet et les moyens femelles pour le couplage sont un deuxième crochet ou une boucle.

16. Source de rayonnement selon les revendications 12 à 14, dans laquelle les moyens mâles pour le couplage comprennent un élément d'espacement et une tête sphérique et la section de réception des moyens femelles pour le couplage est formée par des prolongements des moyens pour le confinement qui définissent un espace creux comportant une cavité pour recevoir l'élément d'espacement lorsque la tête des moyens mâles pour le couplage est disposée dans la partie creuse desdits moyens femelles pour le couplage.

17. Source de rayonnement selon la revendication 1, dans laquelle les germes sont de plus reliés à un fil de transfert.

18. Dispositif pour le traitement par rayonnement endovasculaire, comprenant un cathéter allongé comportant une partie d'extrémité proximale, une partie d'extrémité distale et un conduit s'étendant entre celles-ci pour recevoir une source de rayonnement, et une source de rayonnement selon la revendication 1.

19. Dispositif selon la revendication 18, comprenant une enceinte de confinement pour la source de rayonnement et les germes individuels.

20. Dispositif selon les revendications 18 à 19, comprenant un dispositif de fluoroscopie aux rayons X.

21. Dispositif selon les revendications 18 à 20, comprenant des moyens magnétiques.

22. Dispositif selon les revendications 18 à 21, comprenant de plus un fil de guidage dans un conduit séparé.
